# EUROPEAN PATENT APPLICATION

(11) **EP 4 406 564 A2**
(43) Date of publication of application: **31.07.2024**
(21) Application number: 24175414.2
(22) Date of filing: 16.12.2021
(51) Int. Cl.: A61M 1/06

(54) **BREAST-PUMP SYSTEM**

(30) Priority: 16.12.2020 EP 20214568; 14.10.2021 EP 21202695; 14.10.2021 EP 21202703
(62) Divisional of application: 21823331.0
(71) Applicant: Koninklijke Philips N.V., 5656 AG Eindhoven (NL)
(72) Inventor: VAN VELDHUIZEN, Gijsbert Hendrik, Eindhoven (NL); CLAASSEN, Coen Petrus Martinus, Eindhoven (NL); DOBRUSSKIN, Christoph, Eindhoven (NL); BROCKHUIS, Lili-Marjan, Eindhoven (NL); VAN DEN BIJGAART, Adrianus Wilhelmus Dionisius Maria, Eindhoven (NL)
(74) Representative: Philips Intellectual Property & Standards

(57) **Abstract**

A breast pump system has first and second kits, and they have different sets of functional units. However, they have shape features which are designed to provide similar appearance in use, to make the use of the breast pump system more discrete.

## Description

### FIELD OF THE INVENTION

This invention relates to breast pump systems, in particular with an expression kit for each breast.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted. The breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

Often, it is desirable to express both breasts at the same time. A user will then typically acquire a set of identical devices to achieve a setup which performs stimulation and expression independently for each breast. By having two similar-shaped devices a limited impact on level of discreteness is achieved as the additional volume for each breast is similar. However, this gives a high purchase price and there is no sharing of functions between the two devices.

US 2020/155738 discloses a wearable breast pump. In one example, there are two expression kits, but only one has the controller or battery or pump to be used by both expression kits.

### SUMMARY OF THE INVENTION

The invention is defined by the claims.

According to examples in accordance with an aspect of the invention, there is provided a breast pump system comprising:
a first kit; and
a second kit,
wherein the first and second kits each have a different respective set of functional units which thereby define a different set of functional capabilities of the kit, and the first and second kits have matching outer shapes.

This breast pump system makes use of two kits, but they have different functional capabilities. Thus, the set of functional parts is different between the two kits. In this way, the cost of the overall system can be kept to a minimum by avoiding unnecessary duplication of functions.

The set of functional units of the first and second kit together constitute a drive system for implementing a stimulation mode for triggering the milk ejection reflex and a different expression mode. The set of functional units of the one of the kits (e.g. the second kit) has no simulation mode.

A stimulation and expression function is in this way provided (either in one kit or distributed between the two) but stimulation is provided only at one of the kits. Better value can be achieved, as the price of the overall setup is lower.

A stimulation and expression kit is for example combined with a collection-only or expression-only kit so that better value can be achieved. This may for example be possible by using the MER stimulation for one breast to trigger the MER in both breasts, because it is a hormone based trigger not specific to one breast. However, the solution remains discrete because the outer appearance (in particular the outer shape) of the two devices match. They may for example be worn in a feeding bra with the breast feeding being less obvious to a third party.

The set of functional units of the first kit for example comprises a drive system for implementing both an expression mode and a separate stimulation mode, whereas the set of functional units of the second kit comprises a drive system for implementing an expression mode but no separate stimulation mode.

In one set of examples, the drive system of the first kit comprises a system for generating a pressure waveform for use during a stimulation mode for triggering the milk ejection reflex and a different pressure waveform for use during an expression mode, and the drive system of the second kit comprises a system for generating a pressure waveform for use during an expression mode but no different pressure waveform for use during a stimulation mode for triggering the milk ejection reflex.

These pressure waveforms are for setting a cavity pressure around the nipple and optionally a portion of the breast.

Alternatively, the drive system of the first kit may comprise a system for generating a mechanical contact pressure waveform for use during a stimulation mode for triggering the milk ejection reflex. In this case, the stimulation involves application of a physical contact pressure with the breast.

The differences between the kits may reside in the hardware components and/or the software functionality of a controller.

The first and second kits are designed to avoid a visually perceivable difference in size or shape, i.e. a visually matching outer shape, by having substantially matching outer designs. This means that when in use, the two kits look like a matching pair. They may have identical or mirror symmetrical outer shape. They may have approximately the same internal volume so that the volume added by wearing each kit is the same. For example, the added volume of one kit may be in the range 1.0 to 1.1 times the added volume of the other.

The first and second kits for example have a same cup size for fitting to a feeding bra. The first and second kits may also have the same cup size for fitting to the mother, although it is equally possible for the two kits to have a different (internal) cup size for fitting to the mother but a same external cup size (for mothers with different sized breasts). The two kits for example are intended to sit with the same or similar angle relative to the vertical and project forwardly from the breast by a same or similar amount.

The first and second kits may each have a respective set of functional units, including a different combination of one of more features selected from the list:
a battery;
an electrical pump;
a mechanical system for generating a constant baseline under-pressure;
a system for generating a pressure waveform during a stimulation mode for triggering the milk ejection reflex;
a system for generating a pressure waveform during an expression mode;
a system for generating a pressure waveform during a massage mode;
a milk collection vessel;
a user input interface for receiving user commands;
a user output interface for providing status information to a user;
a communications interface for communicating wirelessly with an external device;
a milk sensor;
a timer; and
an ambient condition sensor,

Each kit is preferably attached to a respective milk collecting vessel. The kit and its respective milk collecting vessel preferably fit in the cup of a breast feeding bra, thus providing a discrete breast-feeding solution.

The system for generating a pressure waveform during a stimulation mode for example comprises a valve and a controller for controlling the valve. This valve is then used in conjunction with an electric vacuum pump to provide a regulated pressure waveform.

The system for generating a pressure waveform during an expression mode may also comprise a valve and a controller for controlling the valve. The control implemented by the controller will be different for the stimulation mode and for the expression mode, for example with different cyclic frequency and optionally also different maximum under-pressure level (i.e. the amount by which the pressure is below atmospheric pressure). Thus, the differences may reside in the control software.

In one example, only one of the first and second kits has a controller for generating the pressure waveform during a stimulation mode. Thus, only one kit is used to stimulate the MER. This creates a hormone response which influences both breasts. Thus, the other kit does not need to have the functionality of the stimulation mode.

In another example, only one of the first and second kits has a user input interface and a user output interface. Thus, any interaction with the user (e.g. advising of battery life, giving information about milk flow etc.) can be implemented in one kit only.

In another example, only one of the first and second kits has an ambient condition sensor. Sensing at only one location is sufficient for many ambient sensing functions. For example, the ambient condition sensor is a noise sensor.

In another example, only one of the first and second kits has a communications interface for communicating wirelessly with an external device. This external device may be a mobile phone which provides an interface to the user.

The first and second kits may for example be coupled together by a wired connection so that the feedback to the user can nevertheless provide status information about both kits.

In another example, only one of the first and second kits has an electrical pump, and the other has a mechanical system for generating a constant baseline under-pressure. This mechanical system for example comprises an element to be squeezed or pressed by the user to generate an under-pressure, which is then retained.

The first and second kits may each be configurable by a user to select a desired set of functional units. This provides a modular system.

One or both kits may have a battery. When only one has a battery, the other may be supplied with power by a wired connection between them.

Another aspect of the invention provides a kit for a breast pump system, comprising:
a housing; and
a set of functional units for mounting to the housing,
wherein at least some of the functional units are modular such that the set of functional units is configurable to implement any desired functionality from the list:
   stimulation and expression;
   stimulation and not expression;
   expression and not stimulation.

The mounting "to" the housing may be in the housing or on the outside of the housing.

The set of functional units is for example configurable to additionally provide massaging functionality and/or local milk collection functionality.

The set of functional units is for example selected from the list:
a battery;
an electrical pump;
a mechanical system for generating a constant baseline under-pressure;
a system for generating a pressure waveform during a stimulation mode for triggering the milk ejection reflex;
a system for generating a pressure waveform during an expression mode;
a system for generating a pressure waveform during a massage mode;
a milk collection vessel;
a user input interface for receiving user commands;
a user output interface for providing status information to a user;
a communications interface for communicating wirelessly with an external device;
a milk sensor;
a timer; and
an ambient condition sensor,

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a mother wearing two wearable breast pumps;
Figure 3 shows a breast pump system in accordance with the invention;
Figure 4 shows one example implementation of the system of Figure 3; and
Figure 5 shows examples of dimensions which enable the use of two similar expression kits to be discrete.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system which has first and second kits, and they have different sets of functional units. They are described below as "expression kits" but in some examples, a kit may not have an expression function. For example, it may simply be a milk collecting vessel, or it may even simply be a part of the hardware of the other (main) kit such as a battery or controller.

The two expression kits have shape features which are designed to provide similar appearance in use, to make the use of the breast pump system more discrete.

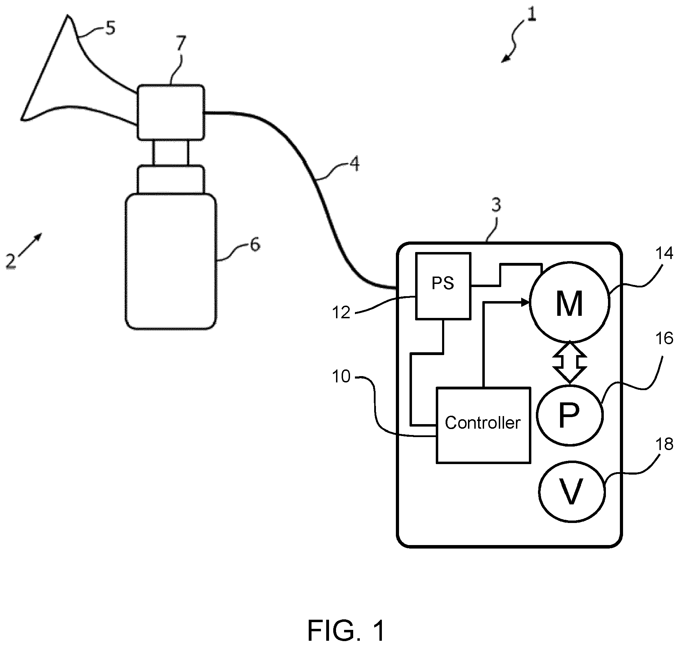

Figure 1 shows a known breast pump system 1, comprising an expression unit 2 and a pump arrangement 3 which are connected via a tube 4. The pump arrangement includes various components in addition to the pump, so may be considered to be a general operating unit.

The expression unit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The controller controls 10 the operation of the power source 12, motor 14 and vacuum pump 16. The pump arrangement 3 further comprises a vacuum release component, such as a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. In one type of design, a top part contains the drivetrain (pump, motor, controller) and a bottom part forms the collection vessel. This enables breast pumping to be performed more discretely. Of course, other designs are possible.

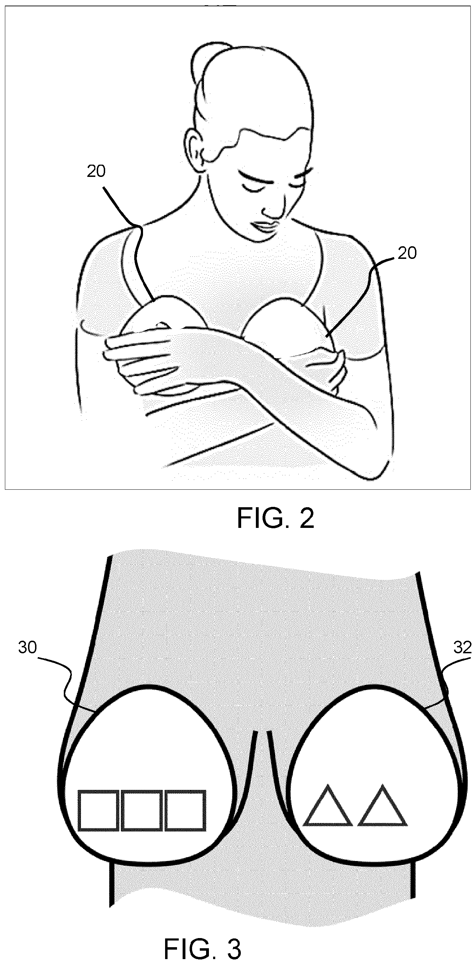

Figure 2 shows a mother wearing two wearable breast pumps 20.

In such a design, the funnel 5 of Figure 1 is implemented as breast shield, and this breast shield is received within an outer enclosure of the wearable breast pump.

Figure 3 shows a breast pump system comprising a first expression kit 30 and a second expression kit 32. Each expression kit is a wearable device, and each expression kit is fitted over a breast shield.

The expression kits may each comprise a milk sensor for sensing milk flow and for measuring flow volume.

The first expression kit 30 has a respective first set of functional units, represented by rectangles. The second expression kit 32 has a respective second set of functional units, represented by triangles. The sets are different although there may be some elements repeated between the sets. For example, each set of functional units includes a breast shield (not shown).

There are various other functional units which can be used to make up the sets.

An expression kit may use electrical pumping, using an electrical pump and motor as explained above, and the pump pressure profile may be controlled by a controller in combination with a pressure regulating valve. Alternatively, a mechanical system may be used for generating a constant baseline under-pressure.

If the milk ejection reflex is triggered in one breast using a particular cyclic pressure waveform during a stimulation mode, the expression of the other breast may simply use a baseline vacuum pressure.

Different controller functions may be implemented, such as the stimulation mode mentioned above and an expression mode using a different cyclic waveform. The controller may also be implemented with a massage mode.

Other functional units include a user input interface for receiving user commands, a user output interface for providing status information to a user, a timer, and a communications interface for communicating wirelessly with an external device such as a mobile telephone. Sensors may also be used, such as a noise sensor or other ambient condition sensors.

A noise sensor is for example used to improve discreetness of the device. For example, the noise level can be reduced (with a consequent decrease in device performance) when a low environmental noise level is detected, in order to maintain discreetness in a smart manner.

The expression kit may include a local milk collection vessel, or it may connect to a remote milk collection vessel. Thus, the expression kit (i.e. the part located over the breast) may include a milk collection vessel or not.

Milk sensing may also be used (in one or both expression kits) to provide feedback to the mother about the milk flow.

As explained above, the first set of functional units is different to the second set of functional units. Thus, the two expression kits have different overall functionality. For example, one may be a master device with a first range of functions, and the other may be a slave device with a reduced second range of functions, wherein functions of the first device which do not need to be duplicated are not present in the second device.

Examples of functions which do not need to be duplicated are:
a battery, because there can be a power connection between the two kits, or the second kit may even be entirely manual;
a controller with a stimulation mode function, because the MER only needs to be triggered in one device;
a user input and/or output interface;
a communication system to a remote device such as a mobile phone;
a pressure regulating valve and associated controller, because the second kit may simply use a static baseline pressure.

The first and second functional units however have matching outer designs.

What is meant by this is that the two expression kits look the same shape when in use. They may have different visual (surface decoration) appearance so the user knows which is which (since they are covered by clothing in normal use), but the form factors (i.e. the shapes but allowing for a possible left-right mirror imaging) are preferably the same, or sufficiently similar that a third party seeing the two kits in use would not perceive a difference in size or shape.

This breast pump system thus makes use of two expression kits, but they have different functional capabilities. In this way, the cost of the overall system can be kept to a minimum by avoiding unnecessary duplication of functions.

The expression kits may have identical shapes, or they may have mirror symmetrical outer shapes (relative to each other). Some the shape parameters which may be designed to equalize and protect discreetness are discussed further below.

One configuration of particular interest is to provide a stimulation and expression kit and a lower cost expression-only kit, so that better value can be achieved. The expression only kit may still have a controller and a pressure regulating valve, but this may be a reduced functionality controller. Instead, the expression only kit may not need to implement a cyclic pressure waveform at all - it may simply implement a constant baseline vacuum. This may be achieved with a pump with no need for a pressure regulating valve, or it may even be achieved with a mechanical system.

When a cyclic waveform is used, different vacuum levels are applied over time. The controller may control a pressure regulating valve, as mentioned above, but it could instead control the drive signal to the pump motor.

When there are cyclic expression and stimulation modes, they for example have different cyclic frequency and different maximum under-pressure (i.e. vacuum) levels. The expression mode for example cycles between a baseline vacuum and a maximum vacuum (i.e. a maximum under-pressure) "Max_Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline_Vac = atmospheric pressure
Max_Vac = 300 mbar (30 kPa) below atmospheric pressure
TTV = 0.85 s
DI = 0.3 s
TTA = 0.05 s
DO = 0.35 s

This gives a total cycle time T_cycle of 1.55s.

These values are simply by way of example. The maximum under-pressure may be higher, for example 350 mbar (35 kPa) and the durations may differ. The cycle time is generally of the order of 1 to 2 seconds.

The stimulation mode for example has a faster frequency hence with a shorter cycle time of around 0.5 seconds, and a lower vacuum, for example a maximum under-pressure level of 150 mbar (15 kPa).

In an expression kit with both stimulation mode and expression modes, the stimulation mode for example takes placing during an initial period of the breast pumping session (e.g. 50 seconds) and the expression mode takes place during the remainder of the breast pumping session.

A controller may also implement a positioning mode. This may comprise intermittent application of a small under-pressure to allow the user to position the breast shield while observing the correct alignment. A controller may also implement a massage mode.

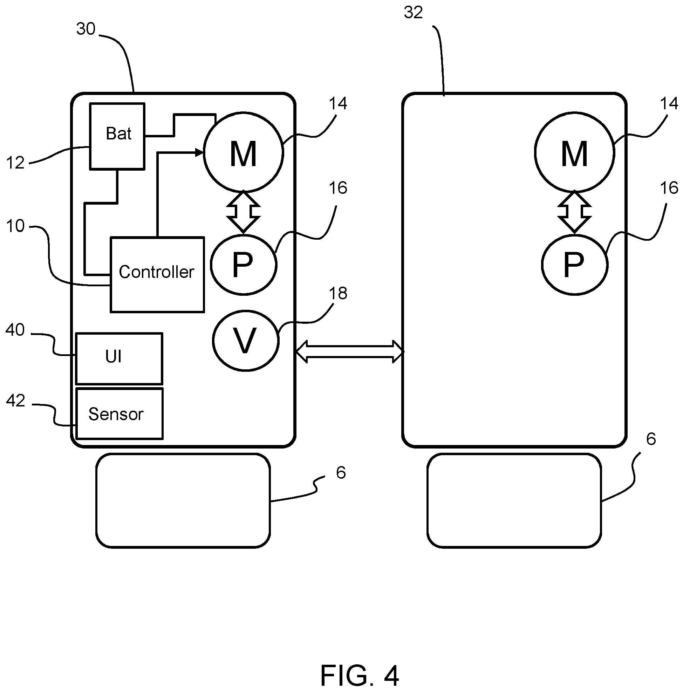

Figure 4 shows an example of an implementation of the system of Figure 3.

A first expression kit 30 has a controller 10, battery 12, motor 14, pump 16 and pressure regulating valve 18. The controller 10 implements a stimulation mode and an expression mode. The first kit 30 also has a user interface 40 (input and output) and a sensor 42.

A second expression kit 30 has motor 14 and pump 16, but power and control commands are received over a wired link to the first kit 30. There is only an expression mode of a constant baseline pressure so no regulating valve is present. The pump motor may, however, be controlled by the first kit to select the baseline pressure level. The second kit is a slimmed down version with no user interface, sensing or power supply. The wired connection does mean however that feedback to the user can nevertheless provide status information about both expression kits.

The second kit may even have a purely mechanical system for generating a constant baseline under-pressure. This mechanical system for example comprises an element to be squeezed or pressed by the user to generate an under-pressure, which is then retained.

The first and second expression kits may each be configurable by a user to select a desired set of functional units. This provides a modular system.

As explained above, the outer shapes are the same or mirror images of each other, for example by having outer shells with a design having similar form factor. The shells may then be used with different functional units as discussed above. A user then buys two or more shells and equips them with different functionality for example to achieve lower cost or for suitability in different use scenarios (e.g. home, work, car etc.).

The first kit in particular may be used on its own. By enabling the functional units to be swapped between shells (which are typically mirror symmetric rather than identical), the full functionality may be fitted to the left shell or the right shell.

In a modular design, the expression kit has a housing and a set of functional units for mounting to (typically in) the housing. The various possible functional units are explained above. At least some of the functional units are modular such that the set of functional units may be selected to implement desired combinations of functions.

As explained above, expression and stimulation are different functions, although the difference may reside in software rather than hardware.

Thus, the modules may be configurable to enable stimulation and expression or stimulation and not expression (in which case the other breast may be expressing) or expression and not stimulation (in which case stimulation may be applied to the other breast).

In further examples, any of the (sixteen) possible combinations of the following functions may configurable:
stimulation functionality;
expression functionality;
massaging functionality; and
local milk collection functionality.

As explained above, the expression kits are designed to be discrete in use, in particular so that a third party will not visually perceive the presence of the breast pump because there is no immediately obvious asymmetry between the two sides.

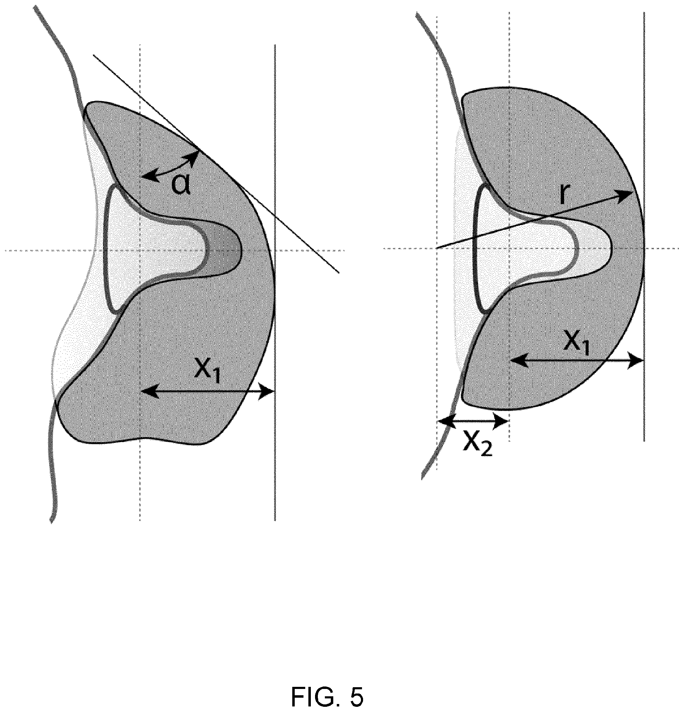

Figure 5 shows some of the design parameters which are important for achieving a matching visual appearance. The left image shows a cross section through a vertical plane (a side view) and the right image shows a cross section through a horizontal plane (a top view).

The dimension X₁ is the amount by which the front of the expression kit extends beyond the front of the breast, i.e. the added thickness.

From above, the front surface is generally circular with radius r. The dimension X₂ is the offset of the center of that circle behind the back of the nipple.

The angle α represent a top surface angle to the vertical.

Each of these parameters may be matched, for example in each case, they may the same, or the larger of the two is in the range of 1.0 to 1.1 times the smaller of the two. The outer surface of each expression kit may correspond to a cup size for fitting to a feeding bra, and the cup sizes are the same. The added volume (i.e. between the breast and the feeding bra) may also be the same, or in the 1.0 to 1.1 range.

In the examples above, the stimulation mode and the expression mode are implemented by applying a pressure waveform. In other examples, the stimulation mode may comprise a mechanical stimulation mode by which mechanical pressure is applied to the breast (i.e. physical contact with the breast applying a force to the breast). The milk ejection reflex can be stimulated by this physical interaction with the breast instead of using the ambient pressure of a cavity around the breast. In such a case, the expression mode may comprise a different mechanical pressure waveform, or it may comprise the application of a base vacuum.

In the examples above, both kits are expression kits, in that they both at least perform an expression function. One kit may in instead only comprise a milk collection vessel, so that it passively collects milk resulting from the stimulation of the milk ejection reflex at the other breast. In such a case, the second kit has no active expression function at all. Other features may be combined with the collection vessel. For example, the second kit may comprise a controller, battery and passive collection vessel, and the first kit is a pump system.

The overall system has different stimulation and expression functions. These may be divided between the two kits, for example with expression only at one side and stimulation only at the other side.

In yet further examples, the second kit does not need to have a milk collection function. For example the first kit may comprise a milk collection vessel container for storing milk expressed through the first kit, and the second kit may then comprise the vacuum pump and an energy storage (battery) for the vacuum pump, and a connection is provided between the first and the second kits to transport the vacuum from the pump to the second kit.

In another example, the first kit comprises a vacuum pump and an energy storage (battery) for the vacuum pump, and the second kit comprises a milk collection vessel container for storing milk expressed through the first kit, and a connection is provided between the first and the second kits to transport the expressed milk from the first kit to the milk collection vessel of the second kit.

In these various ways, the volume necessary to accommodate the components of an integrated breast pump, that are commonly contained in a single housing to be fitted under a bra on a breast, are distributed over two substantially identical housings, that are each to be fitted under a bra on the two breasts of a user.

As explained above, user interface components can again be added to either of the two kit housings, and preferably added to the housing containing the electrical components. The expression funnel and the milk collection vessel are preferably at the same kit so there is no milk channel necessary between the two kits. A smaller milk collection vessel may be provided at the second kit (where the first kit is the one where milk is actively expressed) to catch any milk that may be expressed from the second breast involuntarily.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A kit of parts comprising:
- a breast kit to be positioned on a breast, the kit comprising a housing;
- a set of functional units for mounting to the housing,
**characterized in that**,
the breast kit is user-configurable ,
the functional units are modular to select a desired set from the set of functional units, and
the functional units are configured to be mounted to the housing by a user.

2. The kit of claim 1, wherein the set of functional units is configurable to implement any desired functionality from the list:
stimulation and expression;
stimulation and not expression;
expression and not stimulation.

3. The expression kit of claim 2, wherein the set of functional units is configurable to additionally provide massaging functionality and/or local milk collection functionality.

4. The expression kit of claims 2 or 3, wherein the set of functional units is selected from the list:
a battery (12);
an electrical pump (14, 16);
a mechanical system for generating a constant baseline under-pressure;
a system (12,18) for generating a pressure waveform during a stimulation mode for triggering the milk ejection reflex;
a system (12,18) for generating a pressure waveform during an expression mode;
a system (12,18) for generating a pressure waveform during a massage mode;
a milk collection vessel;
a user input interface (40) for receiving user commands;
a user output interface (40) for providing status information to a user;
a communications interface for communicating wirelessly with an external device;
a milk sensor;
a timer; and
an ambient condition sensor (42).

5. A functional unit for a kit to be positioned on a breast, the kit comprising a housing, and
the functional unit being configured to be mounted to the housing,
**characterized in that**,
the functional unit is configured to be modular and user-selectable.

6. A set of functional units comprising a plurality of functional units as claimed in claim 5.
